(19) Europäisches Patentamt
European Patent Office
Office européen des brevets

(11) **EP 4 443 270 A1**

(12) **EUROPEAN PATENT APPLICATION**
published in accordance with Art. 153(4) EPC

(43) Date of publication:
**09.10.2024 Bulletin 2024/41**

(21) Application number: **22958380.2**

(22) Date of filing: **14.09.2022**

(51) International Patent Classification (IPC):
**G06F 3/01** *(2006.01)*

(52) Cooperative Patent Classification (CPC):
**G06F 3/01**

(86) International application number:
**PCT/CN2022/118674**

(87) International publication number:
**WO 2024/055186 (21.03.2024 Gazette 2024/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Shenzhen Shokz Co., Ltd.**
**Shenzhen, Guangdong 518108 (CN)**

(72) Inventors:
• **LI, Meiqi**
**Shenzhen, Guangdong 518108 (CN)**

• **LIU, Jia**
**Shenzhen, Guangdong 518108 (CN)**
• **SU, Lei**
**Shenzhen, Guangdong 518108 (CN)**
• **ZHOU, Xin**
**Shenzhen, Guangdong 518108 (CN)**
• **LIAO, Fengyun**
**Shenzhen, Guangdong 518108 (CN)**
• **QI, Xin**
**Shenzhen, Guangdong 518108 (CN)**

(74) Representative: **Wang, Bo**
**Panovision IP**
**Ebersberger Straße 3**
**85570 Markt Schwaben (DE)**

(54) **MOTION EVALUATION METHOD AND SYSTEM**

(57)   Embodiments of the present disclosure disclose a motion evaluation method, including: obtaining a motion signal of a subject, the motion signal representing a motion state of the subject; determining an evaluation criterion related to the motion signal; and evaluating the motion signal based on the evaluation criterion.

FIG. 1

EP 4 443 270 A1

# Description

## TECHNICAL FIELD

The present disclosure relates to the technical field of signal detection and evaluation, and in particular, to a motion evaluation method and system.

## BACKGROUND

With people's increasing attention to physical health and scientific motion, motion monitoring devices are vigorously developing. Devices (such as watches, bracelets, etc.) can recognize simple daily motion behaviors (such as running, walking, simple ball games, etc.). After recognizing the type of motion, these devices can provide simple motion statistical parameters (such as running speed, a count of steps walked, a count of hits, etc.) generally after a long period of time. However, the devices cannot determine whether a user's action or motion mode is correct, especially unable to provide real-time feedback to the user.

Since incorrect motion not only fails to achieve the desired fitness effect but may also cause harm to the human body, it is necessary to provide a motion evaluation method that can recognize users' motion errors in real-time and help users correct them, thereby ensuring the scientific motion of the users.

## SUMMARY

The embodiments in the present disclosure provide a motion evaluation method, including: obtaining a motion signal of a subject, the motion signal representing a motion state of the subject; determining an evaluation criterion related to the motion signal; and evaluating the motion signal based on the evaluation criterion.

In some embodiments, the evaluating the motion signal based on the evaluation criterion includes: obtaining an evaluation result by evaluating the motion signal based on the evaluation criterion, the evaluation result including an error type; and generating evaluation feedback based on the evaluation result.

In some embodiments, the generating evaluation feedback based on the evaluation result includes: determining a target feedback mode among a plurality of feedback modes based on the evaluation result or a user type of the subject, wherein the plurality of feedback modes notify the subject at different feedback times or in different feedback types; and generating feedback based on the target feedback mode.

In some embodiments, the motion signal includes at least one of: a posture signal, an electromyography signal, a mechanical signal, an electrocardiography signal, a respiratory signal, or a sweat signal.

In some embodiments, the determining evaluation criterion related to the motion signal includes: determining an action type of the subject by performing an action recognition operation on the subject based on the motion signal; and determining the evaluation criterion related to the motion signal based on the action type.

In some embodiments, the determining an action type of the subject by performing an action recognition operation on the subject based on the motion signal includes: for each frame of the motion signal, determining whether to perform the action recognition operation; and in response to a determination to perform the action recognition operation, determining the action type of the subject by performing the action recognition operation on one or more frames of the motion signal, the one or more frames at least including the frame.

In some embodiments, the determining evaluation criterion related to the motion signal includes: determining a target part corresponding to the action type; and the evaluating the motion signal based on the evaluation criterion includes: obtaining a target motion signal of the target part; and evaluating the target motion signal of the target part based on the evaluation criterion.

In some embodiments, the evaluating the target motion signal of the target part based on the evaluation criterion includes: determining a reference part based on the target part; determining a ratio between an amplitude of the target motion signal of the target part and an amplitude of a reference motion signal of the reference part; determining whether the ratio is less than a ratio threshold; and in response to determining that the ratio is less than the ratio threshold, determining that the evaluation result includes a compensation error.

In some embodiments, the evaluating the target motion signal of the target part based on the evaluation criterion includes: determining an amplitude of the target motion signal; determining whether the amplitude of the target motion signal is less than a first motion amplitude; and in response to determining that the amplitude of the target motion signal is less than the first motion amplitude, determining that the evaluation result includes a compensation error.

In some embodiments, the evaluating the target motion signal of the target part based on the evaluation criterion includes: determining the amplitude of the target motion signal; determining whether the amplitude of the target motion signal is less than a second motion amplitude; and in response to determining that the amplitude of the target motion signal is less than the second motion amplitude, determining that the evaluation result is an efficiency error.

In some embodiments, the target motion signal includes a first signal and a second signal, and the evaluating the target motion signal of the target part based on the evaluation criterion includes: identifying a first feature value of the first signal and a second feature value of the second signal; determining a time difference between the first feature value of the first signal and the second feature value of the second signal; determining whether the time difference is greater than a time difference threshold; and in response to determining that the time difference is greater than the time difference threshold, determining that the evaluation result includes an efficiency error.

In some embodiments, the evaluating the motion signal based on the evaluation criterion includes: determining one or more target parts corresponding to the motion signal based on the evaluation criterion, wherein the one or more target parts include at least two symmetrical parts of the subject; obtaining target motion signals of the at least two symmetrical parts based on the motion signal; and evaluating the target motion signals of the at least two symmetrical parts based on the evaluation criterion.

In some embodiments, the evaluating the target motion signals of the at least two symmetrical parts based on the evaluation criterion includes: determining a signal difference between the target motion signals of the at least two symmetrical parts; determining whether the signal difference is greater than a signal difference threshold; and in response to determining that the signal difference is greater than the signal difference threshold, determining that the evaluation result includes a symmetry error.

In some embodiments, the evaluating the motion signal based on the evaluation criterion includes: determining a target part corresponding to the motion signal based on the evaluation criterion; determining a frequency of a target motion signal of the target part; and determining a fatigue state of the target part based on the frequency and the evaluation criterion.

In some embodiments, the evaluating the motion signal based on the evaluation criterion includes: determining a target part corresponding to the motion signal based on the evaluation criterion; obtaining a target motion signal of the target part; determining an evaluation parameter of the target part based on the target motion signal; and determining an injury type or an injury level of the target part based on the evaluation parameter and the evaluation criterion.

In some embodiments, the evaluation parameter includes at least one of an internal rotation angle, an abduction angle, or a motion acceleration of the target part.

In some embodiments, the method further comprises: evaluating the motion signal based on a motion evaluation model.

The embodiments in the present disclosure provide a method for motion evaluation and feedback, comprising: obtaining a motion signal of a subject, the motion signal representing a motion state of the subject; obtaining an evaluation result by evaluating the motion signal based on an evaluation criterion related to the motion signal; determining a target feedback mode among a plurality of feedback modes based on the evaluation result, wherein the plurality of feedback modes notify the subject at different feedback times or in different feedback types; and generating evaluation feedback based on the target feedback mode.

In some embodiments, the feedback times include immediate feedback or feedback after a motion.

In some embodiments, the feedback types include at least one of: voice feedback, biofeedback, or text feedback.

In some embodiments, the method further comprises: determining an action type of the subject by performing an action recognition operation on the subject based on the motion signal.

In some embodiments, the determining a target feedback mode among a plurality of feedback modes based on the evaluation result includes: determining the target feedback mode among the plurality of feedback modes based on at least one of: the action type, a user type of the subject, and the evaluation result.

In some embodiments, the motion signal includes at least one of: a posture signal, an electromyography signal, a mechanical signal, an electrocardiogram signal, a respiratory signal, or a perspiration signal.

The embodiments in the present disclosure provide a motion evaluation system, including: an acquisition module, configured to obtain a motion signal of a subject, the motion signal representing a motion state of the subject; a determination module, configured to determine an evaluation criterion related to the motion signal; an evaluation module, configured to evaluate the motion signal based on the evaluation criterion.

The embodiments in the present disclosure provide a system for motion evaluation and feedback, including: an acquisition module, configured to obtain a motion signal of a subject, the motion signal representing a motion state of the subject; an evaluation module, configured to obtain an evaluation result by evaluating the motion signal based on evaluation criterion related to the motion signal; a feedback module, configured to determine a target feedback mode among a plurality of feedback modes based on the evaluation result, wherein the plurality of feedback modes notify the subject at different feedback times or in different feedback types; and generate evaluation feedback according to the target feedback method.

The embodiments in the present disclosure provide a computer-readable storage medium, including executable instructions that, when executed by at least one processor, direct the at least one processor to perform the motion evaluation method or the method for motion evaluation and feedback.

Additional features will be partially elaborated in the following description, and will become apparent to those skilled in the art upon reviewing the following content and accompanying drawings, or can be understood through the creation or operation of examples. The features of the present disclosure can be implemented and obtained through practice or by using various aspects of the methods, tools, and combinations elaborated in the following detailed examples.

BRIEF DESCRIPTION OF THE DRAWINGS

This description will be further explained in the form of exemplary embodiments, which will be described in detail by means of accompanying drawings. These embodiments are not restrictive, in which the same numbering indicates the same structure, wherein:

FIG. 1 is a schematic diagram illustrating a motion evaluation system according to some embodiments of the present disclosure;

FIG. 2 is a block diagram illustrating a motion evaluation device according to other embodiments of the present disclosure;

FIG. 3 is a flowchart illustrating a motion evaluation process according to some embodiments of the present disclosure;

FIG. 4 is a flowchart illustrating a process for determining an evaluation criterion according to some embodiments of the present disclosure;

FIG. 5 is a flowchart illustrating a process for evaluating a motion signal according to some embodiments of the present disclosure;

FIG. 6 is a schematic diagram illustrating an electromyography signal and a posture signal of a target part during a bicep curl action according to some embodiments of the present disclosure;

FIG. 7 is a schematic diagram illustrating an electromyography signal and a posture signal of a target part during a seated chest press action according to some embodiments of the present disclosure;

FIG. 8 is a flowchart illustrating another process for evaluating a motion signal according to some embodiments of the present disclosure;

FIG. 9 is a flowchart illustrating yet another process for evaluating a motion signal according to some embodiments of the present disclosure;

FIG. 10A is a schematic diagram illustrating an electromyography signal of a target part during a dumbbell lateral raise action according to some embodiments of the present disclosure;

FIG. 10B is a schematic diagram illustrating an electromyography signal of a target part during a barbell curl action according to some embodiments of the present disclosure;

FIG. 11 is a flowchart illustrating a process for motion evaluation and feedback according to some embodiments of the present disclosure.

**DETAILED DESCRIPTION**

The technical schemes of embodiments of the present disclosure will be more clearly described below, and the accompanying drawings need to be configured in the description of the embodiments will be briefly described below. Obviously, the drawings in the following description are merely some examples or embodiments of the present disclosure, and will be applied to other similar scenarios according to these accompanying drawings without paying creative labor. Unless obviously obtained from the context or the context illustrates otherwise, the same numeral in the drawings refers to the same structure or operation.

As shown in the present disclosure and the patent claims, unless the context clearly suggests exceptions, words such as "a," "an," "one type of," and/or "the" do not specifically refer to the singular and can also include the plural. Generally speaking, the terms "including" and "comprising" only suggest the inclusion of clearly identified steps and elements, and these steps and elements do not constitute an exclusive list. Methods or devices may also contain other steps or elements. The term "based on" means "at least partially based on." The term "one embodiment" represents "at least one embodiment," the term "another embodiment" represents "at least one additional embodiment."

In the description of the present disclosure, it should be understood that the terms "first," "second," etc. are only used for descriptive purposes and should not be interpreted as indicating or implying relative importance or implicitly specifying the number of indicated technical features. Thus, features qualified by "first," "second," etc. may explicitly or implicitly include at least one such feature. In the description of the present disclosure, "a plurality of" means at least two, such as two, three, etc., unless otherwise specifically and clearly defined.

In the field of sports and fitness, there are over 50 common types of fitness action errors. Even for running, there are over 30 common types of errors. As we all know, incorrect action not only fails to achieve the desired fitness effect but may also cause damage to the human body. To address this issue, the embodiments of the present disclosure provide a motion evaluation method and system that may identify users' errors in motion and help them correct the errors, thereby reducing the damage caused to the users during exercise while ensuring the exercise effect.

The following provides a detailed description of the motion evaluation method and system provided in the embodiments of the present disclosure, in combination with the accompanying drawings.

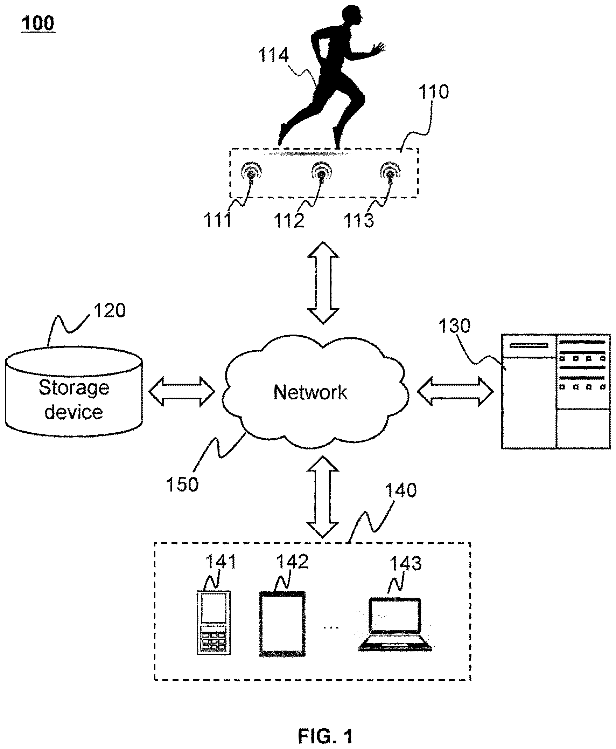

FIG. 1 is a schematic diagram illustrating a motion evaluation system according to some embodiments of the present disclosure.

According to FIG. 1, in some embodiments, a motion evaluation system 100 may include a signal acquisition device 110, a storage device 120, a processing device 130, a terminal device 140, and a network 150. The various components of the motion evaluation system 100 may be connected in a plurality of manners. For example, the signal acquisition device 110 may be connected to the storage device 120 and/or the processing device 130 via the network 150, or may be directly connected to the storage device 120 and/or the processing device 130. As another example, the storage device 120 may be directly connected to the processing device 130 or connected via the network 150. As yet another example, the terminal device 140 may be connected to the storage device 120 and/or the processing device 130 via the network 150, or may be directly connected to the storage device 120 and/or the processing device 130.

The signal acquisition device 110 may collect a motion signal from a subject 114 (e.g., a user) (also referred to a subject 114). The motion signal refers to a signal generated during the motion of the subject 114, which may be used to characterize a motion state of the subject 114. Exemplary motion signals may include a posture signal, an electromyography (EMG) signal, a mechanical signal, an electrocardiography signal, a respiratory signal, a sweat signal, etc. In some embodiments, as shown in FIG. 1, the signal acquisition device 110 may include a posture signal acquisition device 111, an EMG signal acquisition device 112, and a mechanical signal acquisition device 113. In some embodiments, the posture signal acquisition device 111 may include a speed sensor, an inertial sensor (e.g., an acceleration sensor, an angular velocity sensor such as a gyroscope, etc.), an optical sensor (e.g., an optical distance sensor, a video/image collector), an acoustic distance sensor, a tension sensor, etc., or any combination thereof. For example, the signal acquisition device 110 may include a plurality of posture signal acquisition devices 111, which may be provided at different parts of the subject 114 or have different acquisition angles and/or distances relative to the subject 114. In some embodiments, the EMG signal acquisition device 112 may include one or more electrodes. For example, the EMG signal acquisition device 112 may include a plurality of electrodes used to fit different parts of the subject 114 (e.g., chest, back, elbows, legs, abdomen, wrists, etc.) to collect EMG signals from the different parts of the subject 114. In some embodiments, the mechanical signal acquisition device 113 may include a pressure sensor. For example, the pressure sensor may be provided at different parts of the subject 114 to collect pressure signals from the different parts. In some embodiments, mechanical signals of the subject 114 may also be determined based on posture signals and EMG signals. In some embodiments, the signal acquisition device 110 may also include an electrocardiography signal acquisition device, a respiratory signal acquisition device, a sweat signal acquisition device (not shown in FIG. 1), etc. For example, the electrocardiography signal acquisition device may include a plurality of electrodes that may be used to fit different parts of the subject 114 to collect electrocardiography signals from the subject 114. As another example, the respiratory signal acquisition device may include a respiratory rate sensor, a flow sensor, etc., which are used to detect signals such as a respiratory rate and a gas flow of the subject 114 during motion. As yet another example, the sweat signal acquisition device may include a plurality of electrodes that contact the skin of the subject 114, which are used to detect a sweat flow of the subject 114 and analyze sweat components. In some embodiments, the signal acquisition device 110 may have an independent power supply and send collected data to other components in the motion evaluation system 100 (e.g., the storage device 120, the processing device 130, the terminal device 140) via wired or wireless (e.g., Bluetooth, WiFi, etc.) connection.

In some embodiments, the signal acquisition device 110 may send a motion signal of the subject 114 to the storage device 120, the processing device 130, the terminal device 140, etc. via the network 150. In some embodiments, the motion signal collected by the signal acquisition device 110 may be processed by the processing device 130. For example, the processing device 130 may identify an action type of the subject 114 based on the motion signal and evaluate a current action of the subject 114 based on an evaluation criterion related to the action type, thereby obtaining a corresponding evaluation result. As another example, the processing device 130 may directly evaluate the motion signal based on the evaluation criterion related to the motion signal without identifying the action type of the subject. In some embodiments, the evaluation result may be sent to the storage device 120 for recording or to the terminal device 140 for user feedback.

The network 150 may facilitate the exchange of information and/or data. The network 150 may include any suitable network that may facilitate the exchange of information and/or data in the system 100. In some embodiments, at least one component of the motion evaluation system 100 (e.g., the signal acquisition device 110, the storage device 120, the processing device 130, the terminal device 140) may exchange information and/or data with at least one other component of the motion evaluation system 100 via the network 150. For example, the processing device 130 may obtain the motion signal from the signal acquisition device 110 and/or the storage device 120 via the network 150. As another example, the processing device 130 may obtain a user operation instruction from the terminal device 140 via the network 150. Exemplary operation instructions may include but not limited to setting user information (e.g., gender, age, height, weight, disease history, etc.), selecting a motion mode (e.g., running, rope skipping, swimming, muscle training, etc.), and setting a motion time.

In some embodiments, the network 150 may be any form of wired or wireless network, or any combination thereof. For example, the network 150 may include a cable network, a wired network, an optical fiber network, a remote communication network, an internal network, the Internet, a local area network (LAN), a wide area network (WAN), a wireless local area network (WLAN), a metropolitan area network (MAN), a public switched telephone network (PSTN), a Bluetooth network, a ZigBee network, a near-field communication (NFC) network, etc., or any combination thereof. In some embodiments, the network 150 may include at least one network access point, and at least one component of the motion evaluation system 100 may be connected to the network 150 through the access point to exchange data and/or information.

The storage device 120 may store data, instructions, and/or any other information. In some embodiments, the storage device 120 may store data obtained from the signal acquisition device 110, the processing device 130, and/or the terminal device 140. For example, the storage device 120 may store the motion signal collected by the signal acquisition device 110. In some embodiments, the storage device 120 may store data and/or instructions used by the processing device 130 to perform or use the exemplary methods described in the present disclosure. In some embodiments, the storage device 120 may include a mass storage, a removable storage, a volatile read-write memory, a read-only memory (ROM), etc., or any combination thereof. Exemplary mass storages may include disks, optical disks, solid-state disks, etc. In some embodiments, the storage device 120 may be implemented on a cloud platform. For example, the cloud platform may include a private cloud, a public cloud, a hybrid cloud, a community cloud, a distributed cloud, an internal cloud, a multi-layer cloud, etc., or any combination thereof.

In some embodiments, the storage device 120 may be connected to the network 150 to communicate with at least one other component in the motion evaluation system 100 (e.g., the signal acquisition device 110, the processing device 130, the terminal device 140). At least one component in the motion evaluation system 100 may access data, instructions, or other information stored in the storage device 120 via the network 150. In some embodiments, the storage device 120 may be directly connected or communicate with one or more components in the motion evaluation system 100 (e.g., the signal acquisition device 110, the terminal device 140). In some embodiments, the storage device 120 may be part of the signal acquisition device 110 and/or the processing device 130.

The processing device 130 may process data and/or information obtained from the signal acquisition device 110, the storage device 120, the terminal device 140, and/or other components of the motion evaluation system 100. In some embodiments, the processing device 130 may obtain the motion signal of the subject 114 from any one or more of the signal acquisition device 110, the storage device 120, or the terminal device 140, and determine the corresponding action type by processing the motion signal. In some embodiments, the processing device 130 may obtain an evaluation criterion based on the action type corresponding to the motion signal and evaluate the motion signal based on the evaluation criterion. In some embodiments, the processing device 130 may directly determine the evaluation criterion related to the motion signal based on the motion signal and evaluate the motion signal based on the evaluation criterion to obtain an evaluation result. In some embodiments, the processing device 130 may obtain prestored computer instructions from the storage device 120 and execute them to implement the motion evaluation method described in the present disclosure.

In some embodiments, the processing device 130 may be a single server or a server group. The server group may be centralized or distributed. In some embodiments, the processing device 130 may be local or remote. For example, the processing device 130 may access information and/or data from the signal acquisition device 110, the storage device 120, and/or the terminal device 140 via the network 150. As another example, the processing device 130 may be directly connected to the signal acquisition device 110, the storage device 120, and/or the terminal device 140 to access information and/or data. In some embodiments, the processing device 130 may be implemented on a cloud platform. For example, the cloud platform may include a private cloud, a public cloud, a hybrid cloud, a community cloud, a distributed cloud, an inter-cloud cloud, a multi-cloud, etc., or any combination thereof.

The terminal device 140 may receive, send, and/or display data. The received data may include data collected by the signal acquisition device 110, data stored in the storage device 120, evaluation results generated by the processing device 130, etc. For example, the data received and/or displayed by the terminal device 140 may include the motion signal collected by the signal acquisition device 110, the action type of the subject 114 determined by the processing device 130 based on the motion signal, the evaluation criterion determined by the processing device 130, the evaluation result generated by the processing device 130 based on the evaluation criterion, etc. The sent data may include input data and instructions from users (e.g., a fitness coach, a subject to be evaluated). For example, the terminal device 140 may send an operation instruction input by the user to the signal acquisition device 110 via the network 150 to control the signal acquisition device 110 to perform data collection. As another example, the terminal device 140 may send an evaluation instruction input by the user to the processing device 130 via the network 150.

In some embodiments, the terminal device 140 may include a mobile device 141, a tablet computer 142, a laptop 143, etc., or any combination thereof. For example, the mobile device 141 may include a mobile phone, a personal digital assistant (PDA), a medical mobile terminal, etc., or any combination thereof. In some embodiments, the terminal device 140 may include an input device (such as a keyboard, a touch screen), an output device (such as a display, a speaker), etc. In some embodiments, the processing device 130 may be part of the terminal device 140.

It should be noted that the above description of the motion evaluation system 100 is for illustration and explanation purposes only and does not limit the scope of the present disclosure. For those skilled in the art, various modifications and changes can be made to the motion evaluation system 100 under the guidance of the present disclosure. However, these modifications and changes are still within the scope of the present disclosure. For example, the signal acquisition device 110 may include more or fewer components.

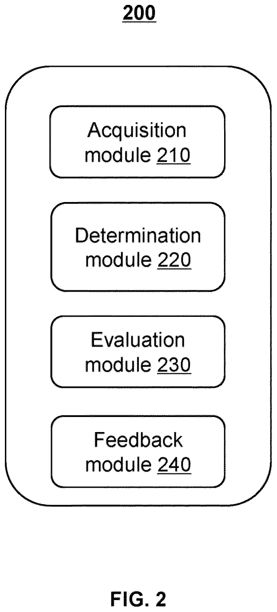

FIG. 2 is a block diagram illustrating a motion evaluation device according to other embodiments of the present disclosure. In some embodiments, the motion evaluation device 200 shown in FIG. 2 may be applied to the motion evaluation system 100 shown in FIG.1 in the form of software and/or hardware. For example, the motion evaluation device 200 may be configured into the processing device 130 and/or the terminal device 140 in the form of software and/or hardware to evaluate the motion signals collected by the signal acquisition device 110.

According to FIG. 2, in some embodiments, the motion evaluation device 200 may include an acquisition module 210, a determination module 220, an evaluation module 230, and a feedback module 240.

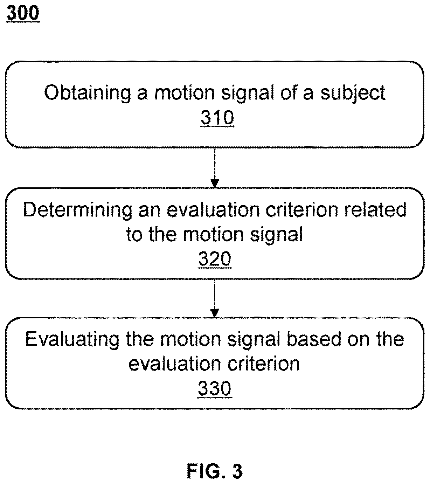

The acquisition module 210 may be configured to obtain a motion signal of the subject 114. For example, the motion signal may be obtained from one or more of the signal acquisition device 110, the storage device 120, and the terminal device 140. In some embodiments, the motion signal may include an EMG signal, a posture signal, a mechanical signal, an electrocardiography signal, a respiratory signal, a sweat signal, etc. More descriptions regarding the motion signal can be found elsewhere in the present disclosure (e.g., FIGs. 1, 3, and relevant descriptions thereof), which is repeated here.

The determination module 220 may be configured to determine an evaluation criterion related to the motion signal. In some embodiments, the determination module 220 may directly determine the evaluation criterion related to the motion signal. In some embodiments, the determination module 220 may determine an action type of the subject 114 by performing an action recognition operation on the subject 114 based on the motion signal, and determine the evaluation criterion related to the motion signal based on the action type. The evaluation criterion may refer to a standard used to evaluate whether a certain action is performed correctly based on the motion signal.

The evaluation module 230 may be configured to evaluate the motion signal of the subject 114 obtained by the acquisition module 210 based on the evaluation criterion determined by the determination module 220, thereby determining an evaluation result of the action performed by the subject 114. For example, the evaluation result may include whether there is an error in the action, a type of the error, a level of the error, etc.

The feedback module 240 may be configured to feed back the evaluation result generated by the evaluation module 230 to a user. In some embodiments, the feedback module 240 may determine the timing of feeding back the evaluation result based on a current action stage and/or a motion scene of the subject 114. In some embodiments, the feedback module 240 may determine a feedback mode and/or the content of the evaluation result based on a user type (e.g., a novice user, an amateur user, a professional user, etc.).

More descriptions regarding each of the above modules can be found elsewhere in the present disclosure (e.g., the sections on FIGs. 3-11 and relevant descriptions thereof), which is not repeated here.

It should be understood that the motion evaluation device 200 and its modules shown in FIG. 2 may be implemented in various manners. For example, in some embodiments, the system and modules may be implemented through hardware, software, or a combination of software and hardware. The hardware may be implemented using dedicated logic; the software may be stored in memory and executed by an appropriate instruction execution system, such as a microprocessor or specially designed hardware. Those skilled in the art can understand that the above methods and systems can be implemented using computer-executable instructions and/or contained in processor control code, for example, such code is provided on a carrier medium such as a disk, CD, or DVD-ROM, a programmable memory such as read-only memory (firmware), or a data carrier such as an optical or electronic signal carrier. The system and modules described in the present disclosure may not only be implemented by hardware circuits such as very large scale integrated circuits or gate arrays, semiconductors such as logic chips and transistors, or programmable hardware devices such as field programmable gate arrays and programmable logic devices, but also may be implemented by software executed by various types of processors, or by a combination of the above hardware circuits and software (e.g., firmware).

It should be noted that the description of the motion evaluation device 200 provided above is for illustrative purposes only and is not intended to limit the scope of the present disclosure. It can be understood that for those skilled in the art, various modules may be arbitrarily combined or formed into subsystems and connected to other modules without departing from this principle based on the description of the present disclosure. For example, the acquisition module 210, the determination module 220, the evaluation module 230, and the feedback module 240 described in FIG. 2 may be different modules in a system, or a single module implementing the functions of two or more of the above modules. As another example, the motion evaluation device 200 may also include a recognition module for performing the action recognition operation on the subject based on the motion signal to determine the action type of the subject. Such variations are within the scope of the present disclosure.

FIG. 3 is a flowchart illustrating a motion evaluation process according to some embodiments of the present disclosure. In some embodiments, the process 300 may be executed through processing logic, which may include hardware (e.g., circuitry, dedicated logic, programmable logic, microcode, etc.), software (instructions executed on processing devices to perform hardware emulation), or any combination thereof. In some embodiments, one or more operations of the motion evaluation process in FIG. 3 may be implemented through the processing device 130 and/or the terminal device 140 illustrated in FIG. 1. For example, the process 300 may be stored in the form of instructions in the storage device 120, and invoked and/or executed by the processing device 130 and/or the terminal device 140. The following describes the execution of the process 300 using the processing device 130 as an example.

According to FIG. 3, in some embodiments, the motion evaluation process 300 may include the following operations.

In 310, a motion signal of a subject may be obtained.

In some embodiments, the motion signal refers to a signal generated by the subject during motion. In some embodiments, the motion signal may characterize a motion state of the subject, including an EMG signal, a posture signal, a mechanical signal, an electrocardiography (ECG) signal, a respiratory signal, a sweat signal, etc., or any combination thereof. In some embodiments, the EMG signal may represent the technical accuracy of the subject's current motion (e.g., a muscle recruitment order) and an injury risk (e.g., a fatigue level). In some embodiments, the EMG signal may be collected through one or more electrodes attached to the subject. For example, a plurality of electrodes may be attached to different parts of the subject (e.g., chest, back, elbows, legs, abdomen, wrists, etc.) to collect the EMG signal from those parts.

The posture signal may include information such as a joint angle, a speed, an acceleration, or a Euler angle, an angular velocity, an angular acceleration, etc. of various parts. In some embodiments, the posture signal may also represent the technical accuracy of the subject's current motion (e.g., a joint angle, a force generation sequence) and an injury risk (e.g., a shoulder impingement). In some embodiments, the posture signal may be collected through a posture signal acquisition device (e.g., the posture signal acquisition device 111 shown in FIG. 1). Exemplary posture signal acquisition devices may include a speed sensor, an inertial sensor (e.g., an acceleration sensor, an angular velocity sensor such as a gyroscope), an optical sensor (e.g., an optical distance sensor, a video/image collector), an acoustic distance sensor, a tension sensor, etc., or any combination thereof.

The mechanical signal refers to a force exerted on the joints of the subject or detected by motion equipment, which may represent an injury risk (e.g., an ankle pressure, a knee pressure). In some embodiments, the mechanical signal may be obtained through a mechanical sensor. For example, the mechanical sensor may include a pressure sensor that obtains pressure signals from different parts of the subject as mechanical signals. In some embodiments, the mechanical signal may be determined based on the posture signal and the EMG signal.

The ECG signal refers to a signal representing the heart activity of the subject. In some embodiments, the ECG signal may be collected through an ECG signal acquisition device. For example, the ECG signal acquisition device may include a plurality of electrodes attached to different parts of the subject to collect the ECG signal. The respiratory signal refers to a signal representing the respiratory state of the subject. In some embodiments, the respiratory signal may be collected through a respiratory signal acquisition device. For example, the respiratory signal acquisition device may include a respiratory rate sensor, a flow sensor, etc., used to detect a respiratory rate, a gas flow, and other data during the subject's motion. The sweat signal refers to a signal representing the sweating condition of the subject. In some embodiments, the sweat signal may be collected through a sweat signal acquisition device. For example, the sweat signal acquisition device may include a plurality of electrodes in contact with the subject's skin to detect a sweat flow or analyze a sweat composition. In some embodiments, the ECG signal, the respiratory signal, the sweat signal, or any combination thereof, may represent the injury risk (e.g., a fatigue level) of the subject's current motion.

In some embodiments, the processing device 130 may directly obtain the motion signal from a signal acquisition device (e.g., the signal acquisition device 110). In some embodiments, the motion signal may be stored in a storage device (e.g., the storage device 120), and the processing device 130 may obtain the motion signal from the storage device.

In 320, an evaluation criterion related to the motion signal may be determined. In some embodiments, operation 320 may be executed by the determination module 220.

In some embodiments, the evaluation criterion refers to a standard used to evaluate whether a certain action is performed correctly based on the motion signal. In some embodiments, the evaluation criterion may include a target part, a target motion signal corresponding to the target part, an evaluation parameter standard corresponding to the target motion signal, etc., or any combination thereof. The target part may refer to a part to be evaluated when a user performs a certain action. The target motion signal may refer to a specific motion signal that needs to be evaluated for the target part, such as one or more of gesture signals, EMG signals, mechanical signals, electrocardiography signals, respiratory signals, sweat signals, etc. The evaluation parameter standard may refer to parameters used to evaluate the target motion signal and corresponding parameter values or ranges of the parameters.

In some embodiments, the evaluation criterion may be used to evaluate the motion signal to determine if there is an error in the motion corresponding to the motion signal and a type of error. In some embodiments, exemplary error types may include an injury error, a compensation error, an efficiency error, a symmetry error, etc., or any combination thereof. The injury error may refer to a motion error that may cause damage to the human body. The compensation error may refer to an error in which a non-target part (such as a muscle) is used to assist in force generation. The efficiency error may refer to that a range of the action is too large or too small when the action is performed in a certain mode such that the target part is in a non-optimal activation state. The symmetry error may refer to an imbalance in force generation between two symmetrical (e.g., bilaterally symmetrical, anteroposteriorly symmetrical) parts of the human body. In some embodiments, for one or more error types, the evaluation result may include a level of the error. For example, an error level of the injury error may include severe, moderate, mild, etc.

In some embodiments, the evaluation criterion may include standards for evaluating different types of errors. For example, for different types of errors, the target parts and/or target motion signals to be evaluated may differ. Correspondingly, the evaluation parameters corresponding to different target motion signals of different target parts may also differ. Thus, when evaluating motion signals, the evaluation criteria may include one or more standards for evaluating one or more error types. In some embodiments, the evaluation criteria may include standards for evaluating preset error types. For example, the user may select the error type to be evaluated through a terminal device (such as the terminal device 140), and the processing device 130 may determine the evaluation criterion based on the error type selected by the user. In some embodiments, the evaluation criteria may include evaluation criteria corresponding to all error types, which are used to evaluate whether there are errors in the motion signals for each error type.

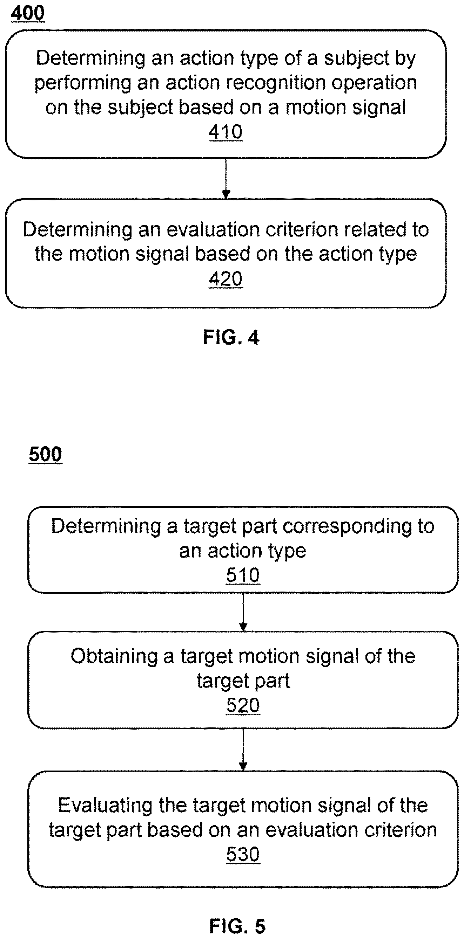

In some embodiments, the evaluation criterion may include a first evaluation criterion that evaluates a first error type related to the action type of the subject. Exemplary first error types may include a compensation error, an efficiency error, etc. When evaluating whether there is the compensatory error or the efficiency error in the subject's motion, the processing device 130 may perform the action recognition operation on the subject based on the motion signal, determine the action type of the subject, and determine the first evaluation criterion related to the motion signal based on the motion type. More descriptions regarding the action recognition operation and the determination of the evaluation criterion can be found elsewhere in the present disclosure (such as FIG. 4 and relevant descriptions thereof), which is not repeated here.

In some embodiments, the evaluation criterion may include a second evaluation criterion that evaluates a second error type that is not related to the action type of the subject. Exemplary second error types may include an injury error, a symmetry error, etc. When evaluating whether there is the injury error or the symmetry error in the subject's motion, the processing device 130 may directly determine the target part and the target signal of the target part in the evaluation criterion, and evaluate the target signal based on the evaluation parameter standard. In some embodiments, the second evaluation criterion may be directly determined based on the motion signal. For example, the evaluation criterion related to the motion signal may be preset, and the processing device 130 may obtain the evaluation criterion and evaluate the motion signal.

In some embodiments, the processing device 130 may also determine the evaluation criterion based on information related to the subject. The information related to the subject may include a gender, an age, a height, a weight, a health state, etc. of the subject. For example, different subjects (such as males and females, adults and minors, healthy individuals and individuals with a history of illness, etc.) may correspond to different evaluation criteria for the same action type.

In some embodiments, the evaluation criterion may be stored in the storage device 120, and the processing device 130 may directly determine the evaluation criterion, or determine the evaluation criterion based on the action type.

In 330, the motion signal may be evaluated based on the evaluation criterion. In some embodiments, operation 330 may be performed by the evaluation module 230.

In some embodiments, the processing device 130 may evaluate the motion signal based on the evaluation criterion to determine the evaluation result. The evaluation result may include whether there is an error in the motion corresponding to the motion signal and the error type. In some embodiments, for one or more error types, the evaluation result may also include an error level of the error type. For example, an error level of an injury error may include severe, moderate, mild, etc. In some embodiments, the processing device 130 may evaluate the motion signal according to a preset evaluation sequence. For example, the processing device 130 may first determine whether there is an injury error in the motion of the subject based on the evaluation criterion, and determine whether there is a compensation error only when it is determined that there is no injury error in the motion. Furthermore, the processing device 130 may determine whether there is an efficiency error only when it is determined that there is no compensation error in the motion of the subject. More descriptions regarding evaluating the motion signal based on the evaluation criterion can be found elsewhere in the present disclosure (e.g., FIGs. 5-9 and relevant descriptions thereof), which is not repeated here.

In some embodiments, after obtaining the evaluation result of the current action performed by the subject, the processing device 130 may also generate evaluation feedback based on the evaluation result. In some embodiments, the processing device 130 may generate the evaluation feedback through various feedback modes, which may notify a user (e.g., a subject to be evaluated or a coach) at different feedback times and/or in feedback types. For example, the feedback times may include immediate feedback or feedback after a motion (e.g., feedback after a single cycle of action, feedback after a single training session, feedback after stopping the movement, etc.). The feedback types may include voice feedback, biofeedback (e.g., electrical stimulation), text feedback, graphical interface feedback, etc., or any combination thereof. In some embodiments, the feedback types may be determined based on a user type of the user who needs feedback. For example, according to the user type, the feedback types may include professional feedback, general feedback, etc. The professional feedback refers to providing feedback to the user in relatively professional language, while the general feedback refers to providing feedback to the user in easy-to-understand language. In some embodiments, the processing device 130 may determine a target feedback mode among a plurality of feedback modes based on the evaluation result, the action type, the user type, etc., or any combination thereof, and provide feedback according to the target feedback mode. For example, the processing device 130 may determine whether there is an injury error in the current action performed by the subject based on the evaluation result. If there is an injury error, the processing device 130 may determine that the feedback time is immediate feedback, and the feedback mode is voice feedback, so that feedback information may be immediately provided to the subject through voice to prevent or reduce the damage suffered by the object during the motion. As another example, if there is no injury error in the current action performed by the subject, but there is another type of error, such as a symmetry error, the processing device 130 may determine that the feedback time is feedback after the motion is finished, and the feedback mode is text and/or graphical interface feedback. As another example, considering that sending the evaluation feedback to the subject may distract the attention of the user, and to avoid increasing risks due to inattention during the motion, when the current action type (e.g., a heavy weight action) performed by the subject is not suitable for immediate feedback, the processing device 130 may determine that the feedback time is feedback after the motion is finished. As another example, when the user type of the subject is a professional fitness person (e.g., a fitness coach), the evaluation result may be displayed through the professional feedback, while when the user is a newcomer to exercise, to facilitate the user's understanding of the evaluation result, the general feedback may be chosen to display the evaluation result.

It should be noted that the above feedback modes are only illustrative examples. In the embodiments of the present disclosure, the target feedback mode may include but is not limited to the above modes. For example, in some embodiments, electrical stimulation may be applied to the part with errors through electrodes to indicate that there are action errors in the corresponding part. As another example, in some embodiments, the processing device 130 may provide the evaluation result to the user according to the feedback mode selected or set by the user. As another example, in some embodiments, when generating the evaluation feedback to the user, the processing device 130 may also show the user a correct motion to guide the user in scientific exercise.

It should be noted that the above process for evaluating the motion signal is only an illustrative example. In some embodiments of the present disclosure, other methods may be used to evaluate the motion signal. For example, the motion signal may be evaluated based on a motion evaluation model. In some embodiments, the motion evaluation model may be a machine learning model obtained through training with several training samples.

It should be noted that the above description of process 300 is for illustration and explanation purposes only and does not limit the scope of the present disclosure. For those skilled in the art, various modifications and changes may be made to process 300 under the guidance of this specification. However, these modifications and changes are still within the scope of the present disclosure. For example, process 300 may also include an operation for recognizing the action of the subject based on a motion signal. As another example, process 300 may also include an operation for generating the evaluation feedback based on the evaluation result.

FIG. 4 is a flowchart illustrating a process for determining an evaluation criterion according to some embodiments of the present disclosure. In some embodiments, the process 400 may be executed through processing logic, which may include hardware (such as circuits, dedicated logic, programmable logic, microcode, etc.), software (instructions running on a processing device to perform hardware simulations), etc., or any combination thereof. In some embodiments, one or more operations in the process 400 illustrated in FIG. 4 may be implemented by the processing device 130 and/or the terminal device 140 illustrated in FIG. 1. For example, the process 400 may be stored in the storage device 120 in the form of instructions and invoked and/or executed by the processing device 130 and/or the terminal device 140. In some embodiments, operation 320 in the motion evaluation process 300 may be implemented through the process 400. In some embodiments, the process 400 may be executed by the determination module 220. The following description takes the execution of the process 400 performed by the processing device 130 as an example.

According to FIG. 4, in some embodiments, the process 400 may include the following operations.

In 410, an action type of the subject may be determined by performing an action recognition operation on the subject based on the motion signal.

The processing device 130 may perform the action recognition operation on the subject based on the motion signal and determine the action type of the subject. In some embodiments, the processing device 130 may determine the action type of the subject based on one or more motion signals collected within a preset time period or continuous motion signals within a preset duration. For example, the processing device 130 may cache 1 to 10 seconds of a continuous motion signal and determine the action type of the subject based on the cached 1 to 10 seconds of continuous motion signal. As another example, the processing device 130 may extract one or more frames of motion signals from the cached 1 to 10 seconds of continuous motion signal and determine the action type of the subject based on the one or more frames of motion signals. In some embodiments, the processing device 130 may collect one frame of motion signal every certain period of time (e.g., 0.5 seconds, 1 second, etc.) after detecting the start of motion of the subject, and determine the action type of the subject based on the collected motion signal.

In some embodiments, to determine the action type of the subject, for each frame of a motion signal, the processing device 130 may determine whether to perform the action recognition operation. In response to a determination to perform the action recognition operation, the processing device 130 may perform the action recognition operation on one or more frames of the motion signal to determine the action type of the subject, the one or more frames at least including the abovementioned frame. For example, in some embodiments, the processing device 130 may determine the action type of the subject based on a posture signal. For each frame of the posture signal, the processing device 130 may determine whether to perform the action recognition operation. For example, for each frame of the posture signal, the processing device 130 may determine whether the signal duration corresponding to the current frame satisfies the preset duration threshold. As another example, for each frame of the posture signal, the processing device 130 may determine whether a count of frames corresponding to the current frame satisfies a preset frame threshold. As another example, for each frame of the posture signal, the processing device 130 may determine whether a difference between the current frame's posture signal and a posture signal of a previous frame (e.g., a first frame, the previous frame, etc.) satisfies a preset difference threshold. The difference between the posture signals may include, for example, a movement distance of the same part of the subject's body in the current frame and the previous frame. Further, in response to the determination to perform the action recognition operation, the processing device 130 may perform the action recognition operation based on one or more frames of the posture signal to determine the action type of the subject. For example, if the count of frames corresponding to the current frame satisfies the preset frame threshold, the processing device 130 may determine to perform the action recognition operation and perform the action recognition operation based on one or more frames of the posture signal.

In some embodiments, the processing device 130 may perform the action recognition operation based on an action recognition model. In some embodiments, an output of the action recognition model may include, but is not limited to, an action type, a count of actions, etc. For example, the action recognition model may determine, based on the motion signal, that a user's action type is seated chest squeeze. In some embodiments, the action recognition model may be a trained machine learning model. In some embodiments, the action recognition model may be trained by the processing device 130 beforehand and stored in the storage device 120, and the processing device 130 may access the storage device 120 to retrieve the action recognition model.

In some embodiments, the action recognition model may be trained based on sample information. The sample information may include a motion signal from a professional (e.g., a fitness coach) and/or a non-professional during exercise. In some embodiments, the motion signal in the sample information may be a processed signal (e.g., segmented, denoised, converted, etc.). In some embodiments, the motion signal may be used as an input to train the machine learning model. In some embodiments, feature information corresponding to the motion signal may be used as an input to train the machine learning model. For example, frequency information and amplitude information of the EMG signal may be used as inputs of the machine learning model. As another example, angular velocity and angular velocity direction/acceleration value of the posture signal may be used as inputs of the machine learning model. As another example, action start points, intermediate points, and end points of motion signals may be used as inputs of the machine learning model. In some embodiments, the machine learning model may include one or more of linear classification models (LR), support vector machine models (SVM), naive Bayes models (NB), K-nearest neighbor models (KNN), decision tree models (DT), ensemble models (RF/GDBT, etc.). In some embodiments, when training the machine learning model to recognize an action type of the user, sample information (each segment of the motion signal) of different action types may be labeled. For example, the sample information of a motion signal generated when a user performs a seated chest squeeze action may be labeled as "1", where "1" represents "seated chest squeeze"; the sample information of a motion signal generated when a user performs a biceps curl action may be labeled as "2", where "2" represents "biceps curl." Feature information of the motion signal (e.g., frequency information and amplitude information of the EMG signal, angular velocity, angular velocity direction, and acceleration value of the posture signal) corresponding to different action types is different. By using labeled sample information as the input to train the machine learning model, an action recognition model for identifying the action type may be obtained. Motion signals and/or corresponding feature information may be input into the machine learning model, and the machine learning model may output the corresponding action type.

In some embodiments, other methods may also be used to determine an action type. For example, the processing device 130 may determine an action type based on a preset rule. Merely by way of example, the firing sequence of relevant muscles differs in different action types, and the preset rule may be a firing sequence of relevant muscles. An action matching database or action matching model may be constructed based on the preset rule. When performing an action type recognition operation, the processing device 130 may determine the muscle firing sequence based on the motion signal and determine the action type based on the action matching database or action matching model. More descriptions regarding the action recognition operation can be found in the international application PCT/CN2021/081931 filed on March 19, 2021, the entire contents of which are incorporated herein by reference.

In some embodiments, to improve the accuracy of action type recognition, the processing device 130 may determine the action type of the subject based on two or more signals among the posture signal, the EMG signal, the mechanical signal, the electrocardiogram signal, the respiratory signal, the sweat signal, etc. For example, a current action type of the subject may be determined based on both the posture signal and the EMG signal. Merely by way of example, for two action types with similar posture signals, EMG signals may be used for action recognition to distinguish between the two different action types. Taking the forward curl and the forward arm extension as examples, the posture signals corresponding to the first half cycle of the forward curl cycle and the second half cycle of the forward arm extension are relatively consistent, while the firing modes of the biceps brachii corresponding to the two actions are different. Therefore, the difference in EMG signals may be used to distinguish the forward curl and the forward arm extension.

In 420, the evaluation criterion related to the motion signal may be determined based on the action type.

In some embodiments, the evaluation criterion may include a target part, a target motion signal corresponding to the target part, an evaluation parameter standard corresponding to the target motion signal, etc., or any combination thereof. In some embodiments, the processing device 130 may determine the target part based on the action type. The target part refers to a key part involved in executing a specific motion. The processing device 130 may determine the key part as the target part for evaluation. In some embodiments, an action type may correspond to one or more target parts. Furthermore, the processing device 130 may obtain the target motion signal of the target part and then evaluate the target motion signal based on the evaluation parameter standard. In some embodiments, the evaluation criterion may be related to an error type. For example, for the same action type, the compensation error and the efficiency error may correspond to different target motion signals. Correspondingly, the evaluation parameter standards for the different target motion signals may also be different. Therefore, after determining the target part, the processing device 130 may perform a motion evaluation based on different evaluation criteria for different types of errors. More descriptions regarding evaluating motion signals based on different types of errors can be found elsewhere in the present disclosure (e.g., FIGs. 5-9 and relevant descriptions thereof), which is not repeated here.

It should be noted that the description of the process 400 above is merely for illustration and explanation purposes and does not limit the scope of the present disclosure. For those skilled in the art, various modifications and changes may be made to the process 400 under the guidance of the present disclosure. However, these modifications and changes still fall within the scope of the present disclosure.

FIG. 5 is a flowchart illustrating a process for evaluating a motion signal according to some embodiments of the present disclosure. In some embodiments, the process 500 may be executed through processing logic, which may include hardware (e.g., circuitry, dedicated logic, programmable logic, microcode, etc.), software (instructions running on a processing device to emulate hardware), etc., or any combination thereof. In some embodiments, one or more operations of the process 500 illustrated in FIG. 5 may be implemented by the processing device 130 and/or the terminal device 140 illustrated in FIG. 1. For example, the process 500 may be stored in the storage device 120 in the form of instructions and invoked and/or executed by the processing device 130 and/or the terminal device 140. In some embodiments, operation 320 and/or operation 330 of the motion evaluation process 300 may be implemented through the process 500. In some embodiments, the process 500 may be executed by the determination module 220 and/or the evaluation module 230. In some embodiments, the motion evaluation related to a first error type (e.g., a compensation error, an efficiency error, etc.) associated with an action type may be achieved through the process 500. The following description takes the execution of the process 500 performed by the processing device 130 as an example.

According to FIG. 5, in some embodiments, the process 500 may include the following operations.

In 510, a target part corresponding to the action type may be determined.

In some embodiments, for different action types, the target part that needs to be evaluated may be different. The processing device 130 may determine the target part based on a target action type and then evaluate a target motion signal of the target part. For example, for an action type such as a seated chest press, the target part may include pectoral muscles.

In 520, a target motion signal of the target part may be obtained.

In some embodiments, the processing device 130 may evaluate the target motion signal corresponding to the target part based on an evaluation criterion. In some embodiments, different target motion signals may be evaluated for different error types. For example, for a compensation error, the target motion signal may include an EMG signal of the target part. As another example, for an efficiency error, the target motion signal may include an EMG signal and/or a posture signal of the target part.

In 530, the target motion signal of the target part may be evaluated based on the evaluation criterion.

In some embodiments, the evaluation criterion may include an evaluation parameter standard corresponding to the target motion signal of the target part. The evaluation parameter standard may refer to parameters used to evaluate the target motion signal and corresponding parameter values or ranges of the parameters.

In some embodiments, the parameters may include a ratio between an amplitude of the motion signal of the target part and an amplitude of a motion signal of a reference part, and the corresponding parameter value or range may include a ratio threshold. The reference part may refer to a part other than the target part. The processing device 130 may determine the ratio between the amplitude of the motion signal of the target part and the amplitude of the motion signal of the reference part, and determine whether the ratio is less than the ratio threshold. If the ratio is less than the ratio threshold, it may indicate that the target part does not exert force properly and non-target parts are used to assist in exerting force. In such cases, an evaluation result of the current motion performed by the subject may be determined as a compensation error. In some embodiments, one or more ratios between the amplitudes of the motion signals of the target parts and the amplitudes of the motion signals of the reference parts when one or more subjects correctly perform the current action type may be obtained, and the ratio threshold may be determined based on the one or more ratios. For example, in the evaluation criterion corresponding to the compensation error, the target motion signal may include an EMG signal of the target part, and the parameters may include a ratio between an amplitude of the EMG signal of the target part and an amplitude of the EMG signal of the reference part. The processing device 130 may determine the ratio between the amplitude of the EMG signal of the target part and the amplitude of the EMG signal of the reference part, and determine whether the ratio is less than the ratio threshold. If the ratio is less than the ratio threshold, the evaluation result of the current motion performed by the subject may be determined as a compensation error.

In some embodiments, the parameters may include the amplitude of the motion signal, and the corresponding parameter values or ranges may include a first motion amplitude. The processing device 130 may determine the amplitude of the motion signal and determine whether the amplitude is less than the first motion amplitude. If the amplitude of the motion signal of the subject is less than the first motion amplitude, it may indicate that the target part does not exert force properly and non-target parts are used to assist in exerting force. In such cases, the evaluation result of the current motion performed by the subject may be determined as a compensation error. In some embodiments, the motion signals of target parts when one or more subjects correctly perform the current action type may be obtained, and a preset motion amplitude may be determined based on the motion signals of the one or more subjects. For example, in the evaluation criterion corresponding to compensation error, the target motion signal may include the EMG signal of the target part, the parameters may include the amplitude of the EMG signal of the target part, and the corresponding parameter values or ranges may include a first EMG amplitude.

In some embodiments, the parameters may include the amplitude of the motion signal of the target part, and the corresponding parameter values or ranges may include a second motion amplitude. The processing device 130 may determine the amplitude of the motion of the target part and determine whether the amplitude of the motion signal is less than the second motion amplitude. If the amplitude is less than the second motion amplitude, it may indicate that the target part has not reached the optimal exercise state. In such cases, the evaluation result of the current motion performed by the subject may be determined as an efficiency error. In some embodiments, the second motion amplitude may be determined based on the amplitudes of motion signals of target parts when one or more subjects correctly perform the current action type. For example, in the evaluation criterion corresponding to the efficiency error, the target motion signal may include an EMG signal or a posture signal of the target part, the parameters may include an amplitude of the EMG signal or the posture signal of the target part, and the corresponding parameter values or ranges may include a second EMG amplitude or a second posture amplitude. In some embodiments, the first motion amplitude and the second motion amplitude may be different values. For example, when evaluating the compensatory error and the efficiency error for the same motion signal (e.g., the EMG signal) of the same target part, the first motion amplitude in the evaluation criterion corresponding to the compensatory error may be less than the second motion amplitude in the evaluation criterion corresponding to the efficiency error. Optionally or additionally, the processing device 130 may first determine whether there is a compensation error in the target part based on the first motion amplitude, and then determine whether there is an efficiency error in the target part based on the second motion amplitude.

In some embodiments, the target motion signal may include a first signal and a second signal, the parameters may include a time difference between a feature value of the first signal and a feature value of the second signal, and the corresponding parameter values or ranges may include a time difference threshold. The processing device 130 may determine the first feature value of the first signal and the second feature value of the second signal, determine the time difference between the first feature value and the second feature value, and determine whether the time difference is greater than the time difference threshold. If the time difference is greater than the time difference threshold, the evaluation result of the current motion performed by the subject may be determined as an efficiency error. The first feature value and the second feature value may refer to feature values in the first signal and the second signal that reflect the motion situation of the subject, such as the maximum and/or minimum amplitude. The time difference between the first feature value and the second feature value may be understood as a difference between a signal acquisition time corresponding to the first feature value and a signal acquisition time corresponding to the second feature value. For example, in the evaluation criterion corresponding to the efficiency error, the first signal may include the EMG signal and the second signal may include the posture signal of the target part, and the parameter may include a time difference between a feature value of the EMG signal and a feature value of the posture signal of the target part.

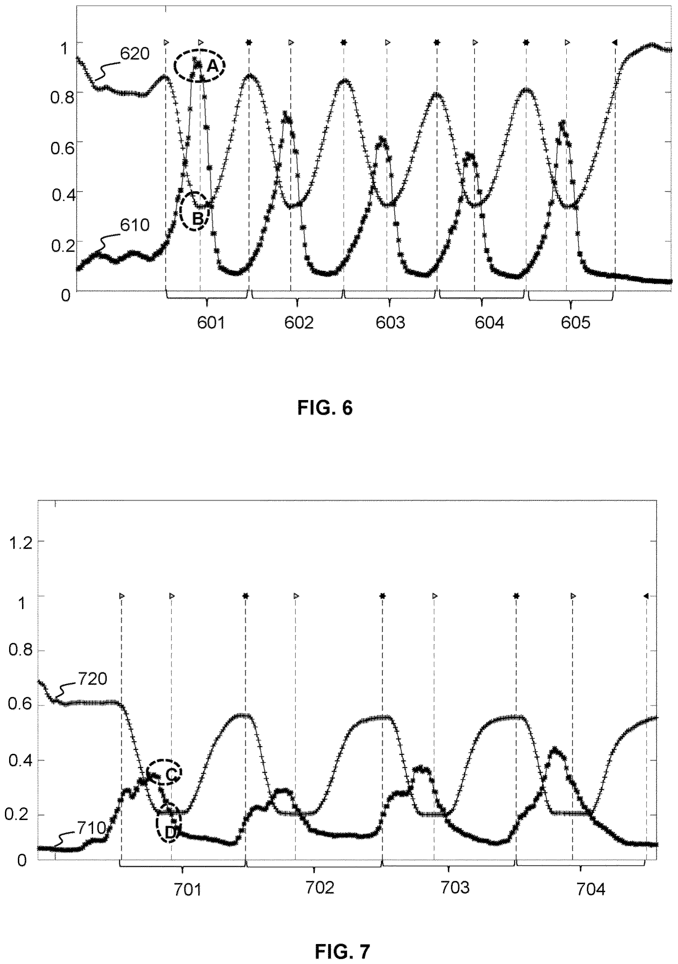

Taking the biceps curl action as an example, FIG. 6 is a schematic diagram illustrating an EMG signal and a posture signal of a target part during a bicep curl action according to some embodiments of the present disclosure. As shown in FIG. 6, the EMG signal of the target part may refer to an EMG signal of the biceps brachii, which may be represented as a curve 610 illustrating the change of the EMG amplitude over time. The posture signal of the target part may be represented by a curve 620 illustrating the change of an angle between an upper arm and a forearm over time, where the amplitude corresponding to the curve 620 may be a normalized value obtained by normalizing the current angle based on the maximum angle between the upper arm and the forearm (i.e., 180°). The horizontal coordinate intervals 601, 602, 603, 604, and 605 shown in FIG. 6 respectively represent the time from the start to the end of the 1 st to 5th sets of biceps curl actions. Optionally or additionally, the time when the action starts may be determined based on the posture signal. Taking a single biceps curl action in interval 601 as an example, the subject started the biceps curl action from a state where the arm is close to straight (i.e., the angle between the upper arm and the forearm is close to 180°). As time increases, the amplitude of the EMG signal of the biceps brachii gradually increases, and the amplitude of the corresponding posture signal gradually decreases. When the amplitude of the EMG signal reaches the maximum value A, it indicates that the biceps brachii has reached the optimal exercise state, and the current biceps curl action may be ended and the next biceps curl action may be started. Correspondingly, during the process of ending the current biceps curl action, the amplitude of the EMG signal gradually decreases, and the arm gradually returns to a nearly straight state, with the amplitude of the corresponding posture signal gradually increasing. In such cases, ending the current biceps curl action when the amplitude of the EMG signal reaches the maximum value may ensure adequate exercise of the target part while avoiding continuing the current action after the target part has reached the optimal exercise state, thus ensuring exercise efficiency. In other words, as shown in FIG. 6, when the time corresponding to the maximum amplitude A of the EMG signal coincides with the time corresponding to the minimum amplitude B of the posture signal, the biceps curl action may achieve optimal exercise efficiency.

Taking the seated chest press action as an example, FIG. 7 is a schematic diagram illustrating an EMG signal and a posture signal of a target part during a seated chest press action according to some embodiments of the present disclosure. As shown in FIG. 7, the EMG signal of the target part may refer to an EMG signal of the chest muscles, which may be represented as a curve 710 illustrating the change of EMG amplitude over time. The posture signal of the target part may be represented as a curve 720 illustrating the change of the angle between the upper arm and the front of the body over time, where the amplitude corresponding to curve 720 may be a normalized value obtained by normalizing the current angle based on the maximum angle (i.e., 180°) between the upper arm and the front of the body. The horizontal coordinate intervals 701, 702, 703, and 704 shown in FIG. 7 respectively represent the time from the start to the end of the 1st to 4th sets of seated chest press actions. Taking a single seated chest press action in interval 701 as an example, the subject started the seated chest press action with the upper arm nearly straight (i.e., the angle between the upper arm and the front of the body is close to 90°). As time increases, the amplitude of the EMG signal of the chest muscles gradually increases, and the amplitude of the corresponding posture signal gradually decreases. When the amplitude of the EMG signal reaches the maximum value C, indicating that the chest muscles have reached the optimal exercise state, the current seated chest press action may be ended and the next seated chest press action may begin. Correspondingly, during the process of ending the current seated chest press, the amplitude of the EMG signal gradually decreases, the upper arm gradually returns to a nearly straight state from a forward state, and the amplitude of the corresponding posture signal gradually increases. In such cases, ending the current seated chest press when the amplitude of the EMG signal reaches the maximum value may fully exercise the target part while avoiding continuing the current action after the target part reaches the optimal exercise state, thus ensuring exercise efficiency. In other words, when the time corresponding to the maximum amplitude C of the EMG signal coincides with the time corresponding to the minimum amplitude D of the posture signal, the seated chest press may achieve optimal exercise efficiency. However, as shown in FIG. 7, the time difference between the time corresponding to the maximum amplitude C of the EMG signal and the time corresponding to the minimum amplitude D of the posture signal is significant, and accordingly, the seated chest press cannot achieve optimal exercise efficiency. In some embodiments, the processing device 130 may determine the time difference between the maximum amplitude of the EMG signal and the maximum amplitude of the posture signal and determine whether the time difference is greater than a threshold value. If the time difference is less than the threshold value, which indicates that the time corresponding to the maximum amplitude of the EMG signal coincides or is close to the time corresponding to the maximum amplitude of the posture signal, the biceps curl action may achieve the optimal or better exercise efficiency. If the time difference is greater than the threshold value, the evaluation result of the current action performed by the subject may be determined as an efficiency error.

It should be noted that the above method of motion evaluation of the first error type related to the action type is merely an illustrative explanation. In some embodiments, other methods may be used for evaluating the first error type. In some embodiments, for the motion evaluation of the compensation error, the processing device 130 may determine a compensation part based on the action type. The compensation part may refer to a part that may compensate during the motion of the action type. For example, in the seated chest squeeze action, the target part may include the pectoralis major, but there may be a compensation error of using the upper trapezius to assist in exerting force. The processing device 130 may determine the upper trapezius as the compensation part and evaluate a motion signal of the compensation part. For example, the processing device 130 may determine whether the amplitude of the EMG signal of the upper trapezius is greater than the preset EMG amplitude corresponding to the upper trapezius. If the amplitude of the EMG signal of the upper trapezius is greater than the preset EMG amplitude corresponding to the upper trapezius, the evaluation result of the current action performed by the subject may be determined as a compensation error. The above embodiment takes the EMG signal as an example for motion evaluation of the compensation error. In some embodiments, the processing device 130 may evaluate the compensation error in motion based on other motion signals. For example, in the seated chest squeeze action, when there is a compensation error of using the upper trapezius to assist in exerting force, the shoulder joint of the subject may be lifted. Therefore, the processing device 130 may determine a lifting angle of the shoulder joint of the subject based on the posture signal and determine whether the lifting angle is greater than an angle threshold (e.g., 15°). If the lifting angle is greater than the angle threshold, the evaluation result of the current action performed by the subject may be determined as a compensation error. As another example, the compensation error may also be evaluated based on other mechanical signals. For example, when there is a compensation error of wrist flipping, the lower palm of the subject may bear more pressure. Therefore, pressure signals from different parts of the palm may be obtained through a pressure sensor, and the processing device 130 may determine whether the subject has a compensation error of wrist flipping based on the pressure signals.

It should be noted that the above description of process 500 is merely for illustration and explanation and does not limit the scope of the present disclosure. For those skilled in the art, various modifications and changes may be made to process 500 under the guidance of the present disclosure. However, these modifications and changes still fall within the scope of the present disclosure. For example, process 500 may also include an operation for evaluating an injury error. The processing device 130 may determine whether there is an injury error in the motion of the subject. If there is not an injury error, the processing device 130 may determine the action type, and determine whether there is a compensation error in the motion of the subject. If there is not a compensation error, the evaluation of the efficiency error may be performed.

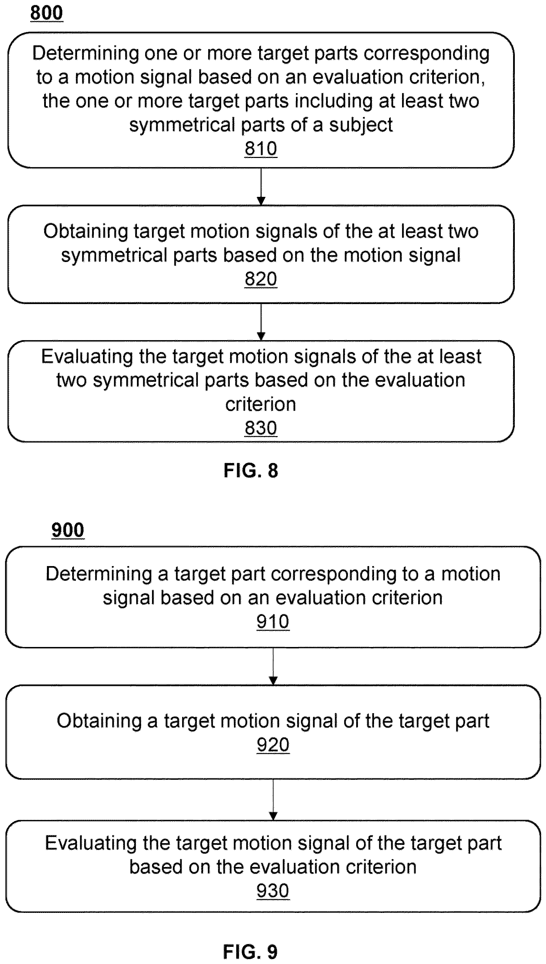

FIG. 8 is a flowchart illustrating another process for evaluating a motion signal according to some embodiments of the present disclosure. In some embodiments, the process 800 may be executed through processing logic, which may include hardware (e.g., circuits, dedicated logic, programmable logic, microcode, etc.), software (instructions running on a processing device to perform hardware simulations), etc., or any combination thereof. In some embodiments, one or more operations in the process 800 illustrated in FIG. 8 may be implemented by the processing device 130 and/or the terminal device 140 illustrated in FIG. 1. For example, the process 800 may be stored in the form of instructions in the storage device 120, and be called and/or executed by the processing device 130 and/or the terminal device 140. In some embodiments, the operation 330 in the motion evaluation process 300 may be implemented through the process 800. In some embodiments, the process 800 may be executed by the evaluation module 230. In some embodiments, motion evaluation for the symmetry error may be performed through the process 800. The following descriptions take the execution the process 800 performed by the processing device 130 as an example.

According to FIG. 8, in some embodiments, the process 800 may include the following operations.

In 810, one or more target parts corresponding to the motion signal may be determined based on the evaluation criterion.

In some embodiments, during the motion evaluation for the symmetry error, the target parts corresponding to the motion signal may be determined based on the evaluation criterion. For example, parts prone to symmetry errors may be determined as the target parts in the evaluation criterion. As another example, any part that generates the motion signal may be determined as the target part in the evaluation criterion. The processing device 130 may determine the target parts corresponding to the motion signal based on the evaluation criterion, thus evaluating the motion signal of the target parts. In some embodiments, the target parts may include at least two symmetrical parts of the subject, such as two parts that are symmetrical from left to right or from front to back.

In 820, target motion signals of the at least two symmetrical parts may be obtained based on the motion signal.

In some embodiments, the processing device 130 may evaluate the target motion signals corresponding to the target parts based on the evaluation criterion. In some embodiments, different target motion signals may be evaluated for different error types. For example, for the symmetry error, the target motion signals may include EMG signals or posture signals of symmetrical parts.

In 830, the target motion signals of the at least two symmetrical parts may be evaluated based on the evaluation criterion.

In some embodiments, the evaluation criterion may include evaluation parameter standards corresponding to the target motion signals of the target parts. The evaluation parameter standards refer to parameters used to evaluate the target motion signals and corresponding parameter values or ranges of the parameters.

In some embodiments, the parameters may include a signal difference between the target motion signals of the symmetrical parts, and the corresponding parameter values or ranges may include a signal difference threshold. The processing device 130 may determine the signal difference between the target motion signals of the symmetrical parts and determine whether the difference is greater than the signal difference threshold. If the difference is greater than the threshold, which indicates that the exercise states of the symmetrical parts are different, the evaluation result of the current action performed by the subject may be determined as a symmetry error. For example, in the evaluation criterion corresponding to the symmetry error, the target motion signals may include EMG signals of symmetrical parts. The parameters may include an amplitude difference between the EMG signals of the symmetrical parts, and the corresponding parameter values or ranges may include an amplitude difference threshold. As another example, in the evaluation criterion corresponding to the symmetry error, the target motion signals may include posture signals of symmetrical parts. The parameters may include a signal difference between the posture signals of the symmetrical parts, and the corresponding parameter values or ranges may include a signal difference threshold. Merely by way of example, the processing device 130 may determine the difference between posture signals (e.g., joint angles) of the symmetrical parts at the same time, and use the difference at a single time or the average of differences at a plurality of times as the signal difference between the posture signals of the symmetrical parts. If the signal difference is greater than the signal difference threshold, which indicates that the exercise states of the symmetrical parts are different, the evaluation result of the current action performed by the subject may be determined as a symmetry error.

In some embodiments, the above thresholds (e.g., the time difference threshold, the amplitude difference threshold, the signal difference threshold, etc.) may be determined in any one or more manners such as historical experience, data statistics, and model predictions.

It should be noted that the above methods for motion evaluation of the symmetry error are merely illustrative. In some embodiments, other methods may be adopted for evaluating symmetry errors in motion, which is not limited in the present disclosure. For example, the symmetry error in motion may also be evaluated based on mechanical signals. Merely by way of example, pressure sensors may be used to obtain pressure signals corresponding to symmetrical parts (e.g., hips, palms of both hands) of the subject, and whether there is a symmetry error (e.g., asymmetric sitting posture, asymmetric force used by both hands) may be determined based on the pressure signals.

It should be noted that the above description of process 800 is merely for illustration and explanation, and does not limit the scope of the present disclosure. For those skilled in the art, various modifications and changes can be made to process 800 under the guidance of the present disclosure. However, these modifications and changes still fall within the scope of the present disclosure. For example, process 800 may further include an operation for determining whether the subject is performing a symmetrical action based on the motion signal. In response to determining that the subject is performing a symmetrical action, the processing device 130 may further evaluate the symmetry error.

FIG. 9 is a flowchart illustrating another process for evaluating a motion signal according to some embodiments of the present disclosure. In some embodiments, the process 900 may be executed by processing logic, which may include hardware (such as circuitry, dedicated logic, programmable logic, microcode, etc.), software (instructions running on processing equipment to perform hardware simulations), etc., or any combination thereof. In some embodiments, one or more operations of the process 900 illustrated in FIG. 9 may be implemented by the processing device 130 and/or the terminal device 140 illustrated in FIG. 1. For example, the process 900 may be stored in the storage device 120 in the form of instructions and invoked and/or executed by the processing device 130 and/or the terminal device 140. In some embodiments, the operation 330 of the motion evaluation process 300 may be implemented through the process 900. In some embodiments, the process 900 may be executed by the evaluation module 230. In some embodiments, the motion evaluation for the injury error may be performed through the process 900. The following descriptions take the execution of the process 900 performed by the processing device 130 as an example.

According to FIG. 9, in some embodiments, the process 900 may include the following operations.

In 910, a target part corresponding to the motion signal may be determined based on the evaluation criterion.

In some embodiments, during the motion evaluation for the injury error, the target part corresponding to the movement signal may be determined based on the evaluation criterion. For example, a part prone to injury error may be determined as the target part in the evaluation criterion. Alternatively, any part that generates the motion signal may be determined as the target part in the evaluation criterion. The processing device 130 may determine the target part corresponding to the motion signal based on the evaluation criterion and evaluate the motion signal of the target part.

In 920, a target motion signal of the target part may be obtained.

In some embodiments, the processing device 130 may evaluate the target motion signal corresponding to the target part based on the evaluation criterion. In some embodiments, for different error types, the target motion signals that need to be evaluated may be different. For example, for the injury error, the target motion signal that needs to be evaluated may include an EMG signal or a posture signal of the target part.

In 930, the target motion signal of the target part may be evaluated based on the evaluation criterion.

In some embodiments, the evaluation criterion may include an evaluation parameter standard corresponding to the target motion signal of the target part. The evaluation parameter standard may refer to parameters used to evaluate the target movement signal and corresponding parameter values or ranges of the parameters.

In some embodiments, the injury error is not related to the motion type performed by the subject, but rather to the subject's body structure and/or motion mode. Exemplary injury errors may include muscle overfatigue, excessively fast joint motion, excessive elbow hyperextension, shoulder impingement, core instability, and so on. For example, when the muscles of the target part are overly fatigued, they may not provide sufficient linking force for the joints, which easily leads to injury. Therefore, the injury error may include a fatigue state. In some embodiments, the parameters in the evaluation criterion corresponding to the fatigue state may include a frequency and/or an amplitude of the target motion signal of the target part, and the corresponding parameter values or ranges may include a frequency-related threshold. The processing device 130 may determine the fatigue state of the target part based on the frequency of the target motion signal and the evaluation criterion. For example, the parameters in the evaluation criterion for fatigue state may include a frequency and/or an amplitude of the EMG signal of the target part, and the corresponding parameter values or ranges may include an EMG frequency-related threshold such as an EMG frequency amplitude, an EMG frequency amplitude slope, and so on. In some embodiments, different degrees of fatigue may correspond to different injury levels. The injury level may indicate the risk or probability of causing injury. Thus, the processing device 130 may determine the injury level based on the degree of fatigue of the target part.

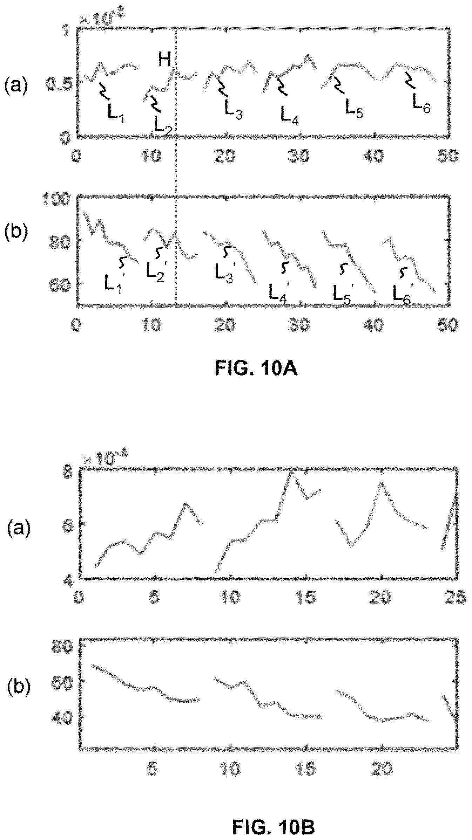

Merely by way of example, FIG. 10A is a schematic diagram illustrating an EMG signal of a target part during a dumbbell lateral raise action according to some embodiments of the present disclosure; FIG. 10B is a schematic diagram illustrating an EMG signal of a target part during a barbell curl action according to some embodiments of the present disclosure. In the dumbbell lateral raise action, the target part may include the deltoid muscle on the left and/or right side of the human body. In the present disclosure, the right deltoid muscle is taken as an example. In FIG. 10A, (a) shows curves illustrating the change of the amplitudes of the EMG signals of the right deltoid muscle over the count of sets of actions, and (b) shows curves illustrating the change of the frequencies of the EMG signals of the right deltoid muscle over the count of sets of actions. $L_1$ represents the amplitude curve of the EMG signal corresponding to the first set of actions, and $L'_1$ represents the frequency curve of the electromyography signal corresponding to the first set of actions; $L_2$ represents the amplitude curve of the EMG signal corresponding to the second set of actions, and $L'_2$ represents the frequency curve of the electromyography signal corresponding to the second set of actions; $L_3$ represents the amplitude curve of the EMG signal corresponding to the third set of actions, and $L'_3$ represents the frequency curve of the electromyography signal corresponding to the third set of actions; $L_4$ represents the amplitude curve of the EMG signal corresponding to the fourth set of actions, and $L'_4$ represents the frequency curve of the electromyography signal corresponding to the fourth set of actions; $L_5$ represents the amplitude curve of the EMG signal corresponding to the fifth set of actions, and $L'_5$ represents the frequency curve of the EMG signal corresponding to the fifth set of actions; $L_6$ represents the amplitude curve of the EMG signal corresponding to the sixth set of actions, and $L'_6$ represents the frequency curve of the EMG signal corresponding to the sixth set of actions. In each set of actions shown in FIG. 10A, the user may continuously perform a plurality of dumbbell lateral raise actions until exhaustion. In the barbell biceps curl action, the target part may include the biceps brachii muscle on the left and/or right side of the human body. The present disclosure takes the right biceps brachii muscle as an example. In FIG. 10B, (a) shows curves illustrating the change of the amplitudes of the EMG signals of the right biceps brachii muscle over the count of sets of action, and (b) shows curves illustrating the change of the frequencies of the EMG signals of the right biceps brachii muscle over the count of sets of actions. Combining the curves in FIG. 10A or FIG. 10B

(e.g., curves $L_2$ and $L'_2$ ), within the same set of actions, as the count of actions increases until exhaustion, the amplitude of the EMG signal begins to decrease after reaching a maximum value H, and the frequency of the EMG signal also decreases significantly. Therefore, injury risk levels may be classified based on the frequency and amplitude of the EMG signal. For example, when the amplitude of the EMG signal begins to decrease after reaching a maximum value, and the frequency of the EMG signal is below 80% of an EMG frequency threshold, it may be determined that there is a risk of primary injury in the current motion; if the frequency of the EMG signal is below 70% of the EMG frequency threshold, it may be determined that there is a risk of moderate injury in the current motion; if the frequency of the EMG signal is below 50% of the EMG frequency threshold, it may be determined that there is a risk of severe injury in the current movement. In some embodiments, the EMG frequency threshold may include a center frequency value of the EMG signal when the target part is in a normal state. In some embodiments, different prompts or suggestions may be provided to the user for different levels of injury risk, such as advising the user to reduce the speed of motion or stop exercising.

In some embodiments, the parameters in the evaluation criterion corresponding to the injury error may include an evaluation parameter related to the motion signal of the target part. For example, the parameters in the evaluation criterion corresponding to the injury error may include an evaluation parameter related to a posture signal of the target part. Exemplary evaluation parameters may include an internal rotation angle, an abduction angle, a motion speed, a motion acceleration, etc., or any combination thereof of the target part. For example, in a lat pulldown action, repeated internal rotation and lifting of the upper arm may lead to repeated impacts between the tendons, ligaments, etc., of the humerus and the upper acromion (the outermost part of the clavicle and scapula), resulting in acromion impingement injuries. In some embodiments, the degree of acromion impingement injury is related to an internal rotation angle, an abduction angle, a lifting angle, a motion speed, a motion acceleration, etc., of the target part. Thus, the acromion impingement injuries may be classified into different injury levels based on different ranges of the internal rotation angle, the abduction angle, the lifting angle, the motion speed, and the motion acceleration of the target part. For example, in the lat pulldown action, taking a position where the arms are raised forward at a 120° angle with the palms facing downward as the initial position, when the internal rotation angle of the upper arm is 0-15° and the duration is greater than a preset duration (e.g., 15 seconds, 30 seconds, etc.), it may be determined that there is a risk of primary injury in the current motion; when the internal rotation angle of the upper arm is 15°-30° and the duration is greater than a preset duration (e.g., 15 seconds, 30 seconds, etc.), it may be determined that there is a risk of moderate injury in the current motion; when the internal rotation angle of the upper arm is greater than 30° and the duration is greater than a preset duration (e.g., 15 seconds, 30 seconds, etc.), it may be determined that there is a risk of severe injury in the current motion. As another example, in a fatigue state, the core stability, action continuity, or action stability of the human body will decrease sharply. A decrease in the core stability may manifest as an increase in the zero-crossing rate of angular velocity in the posture signal of a core part (e.g., the waist); a decrease in the action continuity may manifest as a decrease in the amplitude of the posture signal of the target part, an increase in the action cycle time, etc.; and a decrease in the action stability may manifest as an increase in the zero-crossing rate of angular velocity in the posture signal of the target part, etc. Therefore, the processing device 130 may determine the fatigue state or the injury level of the target part based on the posture signal.

It should be noted that the above description of process 900 is merely for illustration and explanation and does not limit the scope of the present disclosure. For those skilled in the art, various modifications and changes may be made to process 900 under the guidance of the present disclosure. However, these modifications and changes still fall within the scope of the present disclosure. In some embodiments, the injury error may also be evaluated based on other motion signals. Merely by way of example, the parameters in the evaluation criterion corresponding to the injury error (e.g., the fatigue state) may include an evaluation parameter related to an electrocardiogram signal, a respiratory signal, a sweat signal, etc., or any combination thereof of the target part. For example, when a frequency of the electrocardiogram signal of the subject is greater than an electrocardiogram frequency threshold, it may be determined that the subject is in a fatigue state. As another example, when a frequency of the respiratory signal of the subject is greater than a respiratory signal frequency threshold, it may be determined that the subject is in a fatigue state. As yet another example, when the amount of sweat (e.g., sweat per unit time) of the subject determined based on the sweat signal is greater than a sweat threshold, it may be determined that the subject is in a fatigue state.

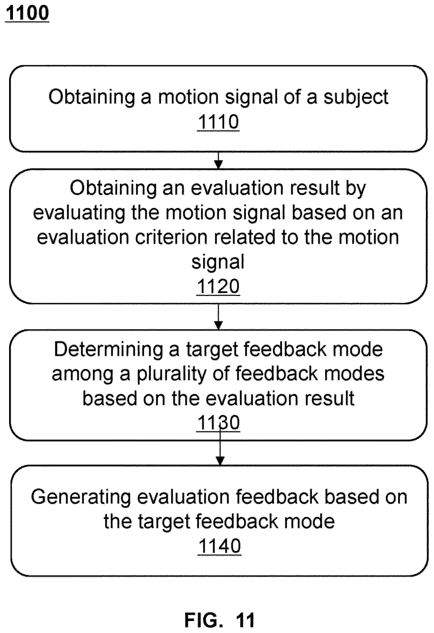

FIG. 11 is a flowchart illustrating a process for motion evaluation and feedback according to some embodiments of the present disclosure. In some embodiments, the process 1100 may be executed by processing logic, which may include hardware (such as circuits, dedicated logic, programmable logic, microcode, etc.), software (instructions running on a processing device to perform hardware simulation), etc., or any combination thereof. In some embodiments, one or more operations in the process 1100 for motion evaluation illustrated in FIG. 11 may be implemented by the processing device 130 and/or the terminal device 140 illustrated in FIG. 1.

According to FIG. 11, in some embodiments, the process 1100 may include the following operations.

In 1110, a motion signal of a subject may be obtained. In some embodiments, operation 1110 may be executed by the acquisition module 210.

According to operation 310, in some embodiments, the motion signal in operation 1110 may refer to a signal generated by the subject during the motion process. In some embodiments, the motion signal may be used to characterize a motion state of the subject, which may include an EMG signal, a posture signal, a mechanical signal, an electrocardiogram signal, a respiratory signal, a sweat signal, etc., or any combination of them. More descriptions regarding obtaining the motion signal can be found in operation 310, which is not repeated here.

In 1120, an evaluation result may be obtained by evaluating the motion signal based on an evaluation criterion related to the motion signal. In some embodiments, operation 1120 may be executed by the evaluation module 230.

In some embodiments, the processing device 130 may directly evaluate the motion signal based on the evaluation criterion related to the motion signal without identifying the action type performed by the subject. For example, the evaluation criterion related to the motion signal may be preset. The processing device 130 may obtain the evaluation criterion and evaluate the motion signal to determine the evaluation result. The evaluation result may include whether there are errors in the motion corresponding to the motion signal and the error type. More descriptions regarding evaluating the motion signal can be found in operation 320-operation330, which is not repeated here.

It should be noted that the above method of evaluating the motion signal is only illustrative. In some embodiments, other evaluation methods may be adopted to evaluate the motion signal of the subject. For example, a motion evaluation model may be used to evaluate the motion signal of the subject. It should be understood that the motion evaluation model may be obtained through a training process using a plurality of training samples. The training samples may include a plurality of sets of motion data and corresponding labels for each set of motion data, where the labels may include error types corresponding to the motion data.

In 1130, a target feedback mode may be determined among a plurality of feedback modes based on the evaluation result.

In 1140, evaluation feedback may be generated based on the target feedback mode.

In some embodiments, operation 1130 and operation 1140 may be executed by the feedback module 240. In some embodiments, the processing device may determine the target feedback mode for presenting the evaluation result from a plurality of feedback modes based on the evaluation result, where the plurality of feedback modes may notify a user at different feedback times or in different feedback types. More descriptions regarding the evaluation feedback can be found elsewhere in operation 330, which is not repeated here.

The potential benefits of the embodiments in the present disclosure include, but are not limited to: (1) By evaluating the motion signal of the subject, the motion errors in the subject's motion may be identified, so as to help the subject correct motion errors, thereby enabling the user to perform a scientific exercise. (2) By identifying the action type of the subject based on the motion signal, determining the evaluation criterion related to the action type, and then evaluating the subject's motion signal based on the evaluation criterion, the accuracy of the evaluation result may be improved. (3) By analyzing the motion signals based on precise categorization of different types of motion errors and referring to motion signals in multiple dimensions, errors in the subject's motion can be determined more accurately, which is more conducive to injury prevention and ability improvement of the user. (4) By determining the target feedback mode for presenting the evaluation result to the user based on the evaluation result, the action type of the subject, and the user type, feedback times and types that better meet user needs can be determined.

It should be noted that different embodiments may produce different beneficial effects. In different embodiments, the beneficial effects may be any one or a combination of several of the above, or any other potentially obtainable beneficial effects.

The basic concepts have been described above, apparently, in detail, as will be described above, and does not constitute limitations of the disclosure. Although there is no clear explanation here, those skilled in the art may make various modifications, improvements, and modifications of present disclosure. This type of modification, improvement, and corrections are recommended in present disclosure, so the modification, improvement, and the amendment remain in the spirit and scope of the exemplary embodiment of the present disclosure.

At the same time, present disclosure uses specific words to describe the embodiments of the present disclosure. As "one embodiment", "an embodiment", and/or "some embodiments" means a certain feature, structure, or characteristic of at least one embodiment of the present disclosure. Therefore, it is emphasized and should be appreciated that two or more references to "an embodiment" or "one embodiment" or "an alternative embodiment" in various parts of present disclosure are not necessarily all referring to the same embodiment. Further, certain features, structures, or features of one or more embodiments of the present disclosure may be combined.

In addition, unless clearly stated in the claims, the order of processing elements and sequences, the use of numbers and letters, or the use of other names in the present disclosure are not used to limit the order of the procedures and methods of the present disclosure. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that the present disclosure object requires more features than the features mentioned in the claims. Rather, claimed subject matter may lie in less than all features of a single foregoing disclosed embodiment.

In some embodiments, the numbers expressing quantities of ingredients, properties, and so forth, used to describe and claim certain embodiments of the application are to be understood as being modified in some instances by the term "about," "approximate," or "substantially". Unless otherwise stated, "about," "approximate," or "substantially" may indicate ±20% variation of the value it describes. Accordingly, in some embodiments, the numerical parameters used in the specification and claims are approximate values, and the approximation may change according to the characteristics required by the individual embodiments. In some embodiments, the numerical parameter should consider the prescribed effective digits and adopt a general digit retention method. Although in some embodiments, the numerical fields and parameters used to confirm the breadth of its range are approximate values, in specific embodiments, such numerical values are set as accurately as possible within the feasible range.

With respect to each patent, patent application, patent application disclosure, and other material cited in the present disclosure, such as articles, books, manuals, publications, documents, etc., the entire contents thereof are hereby incorporated by reference into the present disclosure. Application history documents that are inconsistent with the contents of the present disclosure or that create conflicts are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appended to the present disclosure and those described in the present disclosure, the descriptions, definitions, and/or use of terms in the present disclosure shall prevail.

At last, it should be understood that the embodiments described in the present disclosure are merely illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A motion evaluation method, comprising:

   obtaining a motion signal of a subject, the motion signal representing a motion state of the subject;
   determining an evaluation criterion related to the motion signal; and
   evaluating the motion signal based on the evaluation criterion.

2. The motion evaluation method of claim 1, wherein the evaluating the motion signal based on the evaluation criterion includes:

   obtaining an evaluation result by evaluating the motion signal based on the evaluation criterion, the evaluation result including an error type; and
   generating evaluation feedback based on the evaluation result.

3. The motion evaluation method of claim 2, wherein the generating evaluation feedback based on the evaluation result includes:

   determining a target feedback mode among a plurality of feedback modes based on the evaluation result or a user type of the subject, wherein the plurality of feedback modes notify the subject at different feedback times or in different feedback types; and
   generating feedback based on the target feedback mode.

4. The motion evaluation method of claim 1, wherein the motion signal includes at least one of: a posture signal, an electromyography signal, a mechanical signal, an electrocardiography signal, a respiratory signal, or a sweat signal.

5. The motion evaluation method of claim 1, wherein the determining evaluation criterion related to the motion signal includes:

>  determining an action type of the subject by performing an action recognition operation on the subject based on the motion signal; and
>  determining the evaluation criterion related to the motion signal based on the action type.

6. The motion evaluation method of claim 5, wherein the determining an action type of the subject by performing an action recognition operation on the subject based on the motion signal includes:
for each frame of the motion signal,

>  determining whether to perform the action recognition operation; and
>  in response to a determination to perform the action recognition operation, determining the action type of the subject by performing the action recognition operation on one or more frames of the motion signal, the one or more frames at least including the frame.

7. The motion evaluation method of claim 5, wherein the determining evaluation criterion related to the motion signal includes:

>  determining a target part corresponding to the action type; and the evaluating the motion signal based on the evaluation criterion includes:
>  obtaining a target motion signal of the target part; and
>  evaluating the target motion signal of the target part based on the evaluation criterion.

8. The motion evaluation method of claim 7, wherein the evaluating the target motion signal of the target part based on the evaluation criterion includes:

>  determining a reference part based on the target part;
>  determining a ratio between an amplitude of the target motion signal of the target part and an amplitude of a reference motion signal of the reference part;
>  determining whether the ratio is less than a ratio threshold; and
>  in response to determining that the ratio is less than the ratio threshold, determining that the evaluation result includes a compensation error.

9. The motion evaluation method of claim 7, wherein the evaluating the target motion signal of the target part based on the evaluation criterion includes:

>  determining an amplitude of the target motion signal;
>  determining whether the amplitude of the target motion signal is less than a first motion amplitude; and
>  in response to determining that the amplitude of the target motion signal is less than the first motion amplitude, determining that the evaluation result includes a compensation error.

10. The motion evaluation method of claim 9, wherein the evaluating the target motion signal of the target part based on the evaluation criterion includes:

>  determining the amplitude of the target motion signal;
>  determining whether the amplitude of the target motion signal is less than a second motion amplitude; and
>  in response to determining that the amplitude of the target motion signal is less than the second motion amplitude, determining that the evaluation result is an efficiency error.

11. The motion evaluation method of claim 7, wherein the target motion signal includes a first signal and a second signal, and the evaluating the target motion signal of the target part based on the evaluation criterion includes:

>  identifying a first feature value of the first signal and a second feature value of the second signal;
>  determining a time difference between the first feature value of the first signal and the second feature value of the second signal;
>  determining whether the time difference is greater than a time difference threshold; and
>  in response to determining that the time difference is greater than the time difference threshold, determining that the evaluation result includes an efficiency error.

12. The motion evaluation method of claim 1, wherein the evaluating the motion signal based on the evaluation criterion includes:

>  determining one or more target parts corresponding to the motion signal based on the evaluation criterion, wherein the one or more target parts include at least two symmetrical parts of the subject;
>  obtaining target motion signals of the at least two symmetrical parts based on the motion signal; and
>  evaluating the target motion signals of the at least two symmetrical parts based on the evaluation criterion.

**13.** The motion evaluation method of claim 12, wherein the evaluating the target motion signals of the at least two symmetrical parts based on the evaluation criterion includes:

determining a signal difference between the target motion signals of the at least two symmetrical parts;
determining whether the signal difference is greater than a signal difference threshold; and
in response to determining that the signal difference is greater than the signal difference threshold, determining that the evaluation result includes a symmetry error.

**14.** The motion evaluation method of claim 1, wherein the evaluating the motion signal based on the evaluation criterion includes:

determining a target part corresponding to the motion signal based on the evaluation criterion;
determining a frequency of a target motion signal of the target part; and
determining a fatigue state of the target part based on the frequency and the evaluation criterion.

**15.** The motion evaluation method of claim 1, wherein the evaluating the motion signal based on the evaluation criterion includes:

determining a target part corresponding to the motion signal based on the evaluation criterion;
obtaining a target motion signal of the target part;
determining an evaluation parameter of the target part based on the target motion signal; and
determining an injury type or an injury level of the target part based on the evaluation parameter and the evaluation criterion.

**16.** The motion evaluation method of claim 15, wherein the evaluation parameter includes at least one of an internal rotation angle, an abduction angle, or a motion acceleration of the target part.

**17.** The motion evaluation method of claim 1, further comprising:
evaluating the motion signal based on a motion evaluation model.

**18.** A method for motion evaluation and feedback, comprising:

obtaining a motion signal of a subject, the motion signal representing a motion state of the subj ect;
obtaining an evaluation result by evaluating the motion signal based on an evaluation criterion related to the motion signal;
determining a target feedback mode among a plurality of feedback modes based on the evaluation result, wherein the plurality of feedback modes notify the subject at different feedback times or in different feedback types; and
generating evaluation feedback based on the target feedback mode.

**19.** The method of claim 18, wherein the feedback times include immediate feedback or feedback after a motion.

**20.** The method of claim 18, wherein the feedback types include at least one of: voice feedback, biofeedback, or text feedback.

**21.** The method of claim 18, further comprising:
determining an action type of the subject by performing an action recognition operation on the subject based on the motion signal.

**22.** The method of claim 21, wherein the determining a target feedback mode among a plurality of feedback modes based on the evaluation result includes:
determining the target feedback mode among the plurality of feedback modes based on at least one of: the action type, a user type of the subject, and the evaluation result.

**23.** The method of claim 18, wherein the motion signal includes at least one of: a posture signal, an electromyography signal, a mechanical signal, an electrocardiogram signal, a respiratory signal, or a perspiration signal.

**24.** A motion evaluation system, comprising:

an acquisition module, configured to obtain a motion signal of a subject, the motion signal representing a motion state of the subject;
a determination module, configured to determine an evaluation criterion related to the motion signal;
an evaluation module, configured to evaluate the motion signal based on the evaluation criterion.

**25.** A system for motion evaluation and feedback, comprising:

an acquisition module, configured to obtain a motion signal of a subject, the motion signal representing a motion state of the subject;
an evaluation module, configured to obtain an evaluation result by evaluating the motion signal based on evaluation criterion related to the motion signal;
a feedback module, configured to

determine a target feedback mode among a plurality of feedback modes based on the evaluation result, wherein the plurality of feedback modes notify the subject at different feedback times or in different feedback types; and
generate evaluation feedback according to the target feedback method.

26. A computer-readable storage medium, comprising executable instructions that, when executed by at least one processor, directs the at least one processor to perform the method according to any one of claims 1 to 23.

100

114

110

111    112    113

120

Storage
device

Network

130

150

140

141    142    143

FIG. 1

**200**

Acquisition
module 210

Determination
module 220

Evaluation
module 230

Feedback
module 240

**FIG. 2**

<u>**300**</u>

Obtaining a motion signal of a subject
<u>310</u>

Determining an evaluation criterion related
to the motion signal
<u>320</u>

Evaluating the motion signal based on the
evaluation criterion
<u>330</u>

**FIG. 3**

**400**

Determining an action type of a subject by performing an action recognition operation on the subject based on a motion signal
410

Determining an evaluation criterion related to the motion signal based on the action type
420

**FIG. 4**

**500**

Determining a target part corresponding to an action type
510

Obtaining a target motion signal of the target part
520

Evaluating the target motion signal of the target part based on an evaluation criterion
530

**FIG. 5**

**FIG. 6**

**FIG. 7**

<u>800</u>

Determining one or more target parts corresponding to a motion signal based on an evaluation criterion, the one or more target parts including at least two symmetrical parts of a subject
<u>810</u>

↓

Obtaining target motion signals of the at least two symmetrical parts based on the motion signal
<u>820</u>

↓

Evaluating the target motion signals of the at least two symmetrical parts based on the evaluation criterion
<u>830</u>

**FIG. 8**

<u>900</u>

Determining a target part corresponding to a motion signal based on an evaluation criterion
<u>910</u>

↓

Obtaining a target motion signal of the target part
<u>920</u>

↓

Evaluating the target motion signal of the target part based on the evaluation criterion
<u>930</u>

**FIG. 9**

**FIG. 10A**

**FIG. 10B**

**1100**

Obtaining a motion signal of a subject
1110

Obtaining an evaluation result by evaluating the motion signal based on an evaluation criterion related to the motion signal
1120

Determining a target feedback mode among a plurality of feedback modes based on the evaluation result
1130

Generating evaluation feedback based on the target feedback mode
1140

FIG. 11

## EP 4 443 270 A1

| | INTERNATIONAL SEARCH REPORT | International application No. |
|---|---|---|
| | | **PCT/CN2022/118674** |

**A. CLASSIFICATION OF SUBJECT MATTER**

G06F3/01(2006.01)i

According to International Patent Classification (IPC) or to both national classification and IPC

**B. FIELDS SEARCHED**

Minimum documentation searched (classification system followed by classification symbols)

G06F(IPC)

Documentation searched other than minimum documentation to the extent that such documents are included in the fields searched

Electronic data base consulted during the international search (name of data base and, where practicable, search terms used)

CNABS, WPABS, WPABSC, VCN, DWPI, CNTXT, ENTXT, ENTXTC, CNKI, CSDN: 运动, 健身, 锻炼, 姿势, 姿态, 动作, 标准, 错误, 评估, 打分, 类型, 种类, 类别, 反馈, 时间, 振动, 提醒, 频率, 幅值, 幅度, 速度, exercise, fitness, posture, motion, criteria, error, assessment, score, type, category, feedback, time, vibration, reminder, show, frequency, amplitude, speed.

**C. DOCUMENTS CONSIDERED TO BE RELEVANT**

| Category* | Citation of document, with indication, where appropriate, of the relevant passages | Relevant to claim No. |
|---|---|---|
| X | CN 110471529 A (BEIJING CALORIE TECHNOLOGY CO., LTD.) 19 November 2019 (2019-11-19)<br>description, paragraphs 2-4 and 32-133 | 1-26 |
| A | CN 109864741 A (KINGFAR INTERNATIONAL INC.) 11 June 2019 (2019-06-11)<br>entire document | 1-26 |
| A | CN 110502395 A (STATE GRID SHANXI ELECTRIC POWER CO., LTD.) 26 November 2019 (2019-11-26)<br>entire document | 1-26 |
| A | WO 2014145711 A1 (MOVEMENT TRAINING SYSTEMS LLC) 18 September 2014 (2014-09-18)<br>entire document | 1-26 |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **18 May 2023** | **22 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2022/118674**

| Patent document cited in search report | | | Publication date (day/month/year) | Patent family member(s) | | | Publication date (day/month/year) |
|---|---|---|---|---|---|---|---|
| CN | 110471529 | A | 19 November 2019 | None | | | |
| CN | 109864741 | A | 11 June 2019 | None | | | |
| CN | 110502395 | A | 26 November 2019 | None | | | |
| WO | 2014145711 | A1 | 18 September 2014 | AU | 2014232710 | A1 | 12 November 2015 |
| | | | | GB | 201518100 | D0 | 25 November 2015 |
| | | | | GB | 2527460 | A | 23 December 2015 |
| | | | | GB | 2527460 | B | 14 February 2018 |
| | | | | CA | 2909514 | A1 | 18 September 2014 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2021081931 W